(19) Europäisches Patentamt
European Patent Office
Office européen des brevets

(11) Veröffentlichungsnummer: 0 045 892
B1

# (12) EUROPÄISCHE PATENTSCHRIFT

(45) Veröffentlichungstag der Patentschrift: **01.02.84**

(51) Int. Cl.³: **C 07 D 263/58,** C 08 G 18/28, C 08 G 18/38, C 08 G 73/00, C 08 G 73/22

(21) Anmeldenummer: **81105907.0**

(22) Anmeldetag: **27.07.81**

## (54) Verfahren zur Herstellung von Oxazolin-2-on-Ringe aufweisenden Kunststoffvorläufern und ihre Verwendung zur Herstellung von hochmolekularen Kunststoffen.

(30) Priorität: **07.08.80 DE 3029922**

(43) Veröffentlichungstag der Anmeldung: **17.02.82 Patentblatt 82/7**

(45) Bekanntmachung des Hinweises auf die Patenterteilung: **01.02.84 Patentblatt 84/5**

(84) Benannte Vertragsstaaten: **BE DE FR GB IT**

(56) Entgegenhaltungen:
**DE - A - 2 012 809**

**Chemical Abstracts, Band 88, Nr. 5, 30. Januar 1978 Columbus, Ohio, USA J.F. KINSTLE et al. "Synthesis and characterization of linear and crosslinked Ab-type polyurethanes" Seite 17, Abstract Nr. 38384h**

(73) Patentinhaber: **BAYER AG, Zentralbereich Patente, Marken und Lizenzen, D-5090 Leverkusen 1, Bayerwerk (DE)**

(72) Erfinder: **Rasshofer, Werner, Dr., Wolfskaul 10, D-5000 Köln 80 (DE)**
Erfinder: **Grögler, Gerhard, Dr., von-Diergardt-Strasse 46, D-5090 Leverkusen (DE)**
Erfinder: **Meyborg, Holger, Dr., Bergisch-Gladbacher-Strasse 1, D-5068 Odenthal (DE)**

EP 0 045 892 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

BUNDESDRUCKEREI BERLIN

Verfahren zur Herstellung von Oxazolin-2-on-Ringe aufweisenden Kunststoffvorläufern und ihre Verwendung zur Herstellung von hoch hochmolekularen Kunststoffen

Die vorliegende Erfindung betrifft ein neues Verfahren zur Herstellung von Oxazolin-2-on-Ringe aufweisenden Kunststoffvorläufern durch Umsetzung von bestimmten, nachstehend näher beschriebenen Aminen mit mindestens einem ankondensierten Oxazolin-2-on-Ring mit, gegenüber den aromatischen Aminogruppen im Sinne einer Additions- oder Kondensationsreaktion reaktionsfähige Gruppen aufweisenden Kunststoffvorläufern, sowie die Verwendung der erfindungsgemäßen Verfahrensprodukte zur Herstellung von hochmolekularen Kunststoffen.

Es ist bereits bekannt, Monooxazoline mit Anhydriden von aliphatischen Monocarbonsäuren bei Rückflußtemperaturen zu erhitzen, anschließend mit Wasser zu behandeln, um unter Ringöffnung der Oxazoline zu den entsprechenden O-acylierten Carbonsäureamiden zu gelangen (vgl. US-PS 2 410 318). Weiter ist bekannt, beispielsweise 2-Phenyloxazolin mit p-Nitrobenzoesäure durch Erhitzen zum entsprechenden $\beta$-Benzamidoethyl-p-nitrobenzoat umzusetzen (vgl. Journal of Organic Chemistry, Band 15, 1950, Seiten 802–806). Weiterhin ist bekannt, Polyoxazoline mit 2 oder mehr Oxazolinringen zusammen mit Produkten, die ein mittleres Molekulargewicht von mindestens 600 besitzen und 2 oder mehr Carboxylgruppen als einzige mit den Oxazolinringen reaktionsfähigen Gruppen enthalten, bei 100–300°C zu Carbonsäureester- und Carbonsäureamidgruppen enthaltenden, vernetzten, hochmolekularen Stoffen umzusetzen (vgl. DE-OS 2 012 809). Schließlich war es bereits bekannt, daß cyclische Benzoxazinone bei höheren Temperaturen zu vernetzten Materialien umgesetzt werden können, welche Harnstoff-, Isocyanurat-, Diarylmethan- und Carbodimid-Einheiten aufweisen. Alle diese vorbekannten Verfahren können jedoch mit dem nachstehend näher beschriebenen erfindungsgemäßen Verfahren in keinen näheren Zusammenhang gebracht werden, da die dem erfindungsgemäßen Verfahren zugrunde liegende Reaktion in einer Additions- oder Kondensationsreaktion der Aminogruppen aromatischer, mindestens einen ankondensierten Oxazolin-2-on-Ring aufweisender aromatischer Amine besteht.

Beim nachstehend näher beschriebenen erfindungsgemäßen Verfahren und bei der erfindungsgemäßen Verwendung der erfindungsgemäßen Verfahrensprodukte bedient man sich des Umstands, daß aromatische Amine, welche mindestens einen ankondensierten Oxazolin-2-on-Ring aufweisen, Verbindungen darstellen, die unterschiedliche Reaktivzentren mit einer unterschiedlichen Reaktionsbereitschaft gegenüber zu Additions- oder Kondensationsreaktionen befähigten Verbindungen aufweisen.

Gegenstand der vorliegenden Erfindung ist ein Verfahren zur Herstellung von unter dem Einfluß von Hitze und gegebenenfalls in Gegenwart von Kettenverlängerungs- bzw. Vernetzungsmitteln zu hochmolekularen, gegebenenfalls vernetzten Kunststoffen ausreagierenden, Oxazolin-2-on-Ringe aufweisenden Kunststoffvorläufern, dadurch gekennzeichnet, daß man mindestens zwei mit primären oder sekundären, aromatisch gebundenen Aminogruppen im Sinne einer Additions- oder Kondensationsreaktion reaktionsfähige Gruppierungen aufweisende Kunststoffvorläufer, ausgewählt aus der Gruppe bestehend aus organischen Polyisocyanaten, Polyepoxiden, Carbonsäurechloriden mit mindestens zwei Säurechloridgruppen, Carbonsäureestern mit mindestens zwei Estergruppen und Carbonsäureanhydriden mit mindestens zwei Anhydridgruppen, mit mindestens einen ankondensierten Oxazolin-2-on-Ring aufweisenden aromatischen Aminen mit mindestens einer primären oder sekundären aromatisch gebundenen Aminogruppe der allgemeinen Formel

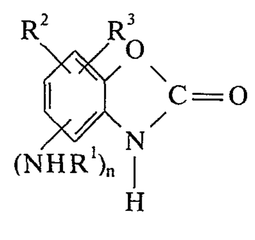

in welcher

$R^1$ für Wasserstoff oder einen aliphatischen Kohlenwasserstoffrest mit 1–4 Kohlenstoffatomen steht,

$R^2$ und $R^3$ entweder für gleiche oder verschiedene Reste stehen und Wasserstoff, einen aliphatischen Kohlenwasserstoffrest mit 1–4 Kohlenstoffatomen oder Chlor bedeuten oder gemeinsam für einen ankondensierten, gegebenenfalls mit einer Aminogruppe $-NHR^1$ substituierten Benzolring stehen oder wobei $R^2$ die eingangs genannte Bedeutung hat und $R^3$ für einen Rest der Formel

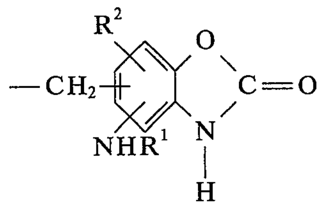

steht, und

n für 1 steht bzw. im Falle des Vorliegens eines aminosubstituierten, ankondensierten aromatischen Rings auch für 0 stehen kann, im Sinne einer Additions- oder Kondensationsreaktion umsetzt, wobei man die Mengen der Reaktionspartner so wählt, daß auf jede aromatisch gebundene primäre oder sekundäre Aminogruppe 0,95 bis 5 an mit diesen Aminogruppen reaktionsfähige Gruppen des Kunststoffvorläufers entfallen.

Gegenstand der vorliegenden Erfindung ist auch die Verwendung der nach diesem Verfahren erhältlichen, Oxazolin-2-on-Ringe aufweisenden Kunststoffvorläufer zur Herstellung von hochmolekularen Kunststoffen, dadurch gekennzeichnet, daß man die Kunststoffvorläufer gegebenenfalls in Gegenwart von Verbindungen, die mindestens zwei, mit Oxazolin-2-on-Ringen im Sinne einer Additions oder Kondensationsreaktion reaktionsfähigen Gruppen einer Hitzebehandlung im Bereich von 100 bis 200° C unterwirft.

Beim erfindungsgemäßen Verfahren werden aromatische Amine mit ankondensiertem Oxazolin-2-on-Ring der Formel

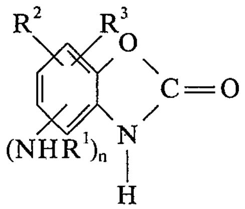

eingesetzt, wobei

$R^1$ für Wasserstoff oder einen aliphatischen Kohlenwasserstoffrest mit 1–4 Kohlenstoffatomen steht,

$R^2$ und $R^3$ entweder für gleiche oder verschiedene Reste stehen und Wasserstoff, einen aliphatischen Kohlenwasserstoffrest mit 1–4 Kohlenstoffatomen oder Chlor bedeuten oder gemeinsam für einen ankondensierten, gegebenenfalls mit einer Aminogruppe $-NHR^1$ substituierten Benzolring stehen oder wobei $R^2$ die eingangs genannte Bedeutung hat und $R^3$ für einen Rest der Formel

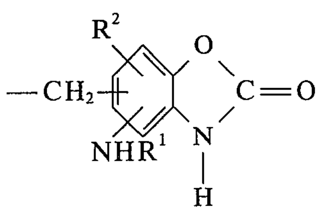

steht, und

n für 1 steht bzw. im Falle des Vorliegens eines aminosubstituierten, ankondensierten aromatischen Rings auch für 0 stehen kann.

Besonders bevorzugt werden solche Verbindungen der genannten allgemeinen Formel eingesetzt, für welche

$R^1$ für Wasserstoff steht,

$R^2$ und $R^3$ für gleiche oder verschiedene Reste stehen und Wasserstoff, einen aliphatischen Kohlenwasserstoffrest mit 1–4 Kohlenstoffatomen oder Chlor, insbesondere jedoch Wasserstoff bedeuten und

n für die Zahl 1 steht.

Typische Beispiele aromatischer Amine mit ankondensiertem Oxazolin-2-on-Ring sind 4-Aminobenzoxazolin-2-on, 5-Aminobenzoxazolin-2-on, 6-Aminobenzoxazolin-2-on, 7-Aminobenzoxazolin-2-on,

4-Amino-6-methylbenzoxazolin-2-on, 5-Amino-6-methyl-benzoxazolin-2-on, 5-Amino-6-chlorbenzoxazolin-2-on, 9-Aminobenzo(g)benzoxazolin-2-on, 5-Aminobenzo(g)benzoxazolin-2-on, 4-Aminobenzo(e)benzoxazolin-2-on, 8-Aminobenzo(f)benzoxazolin-2-on, 10-Aminonaphthaleno(g)benzoxazolin-2-on, Bis(4-aminobenzoxazolin-2-on-6-yl)-methan, Bis(5-aminobenzoxazolin-2-on-6-yl)methan, Bis(7-aminobenzoxazolin-2-on-6-yl)-methan, Bis(4-aminobenzoxazolin-2-on-5-yl)-methan, Bis(6-aminobenzoxazolin-2-on-5-yl)-methan, Bis(7-aminobenzoxazolin-2-on-5-yl)-methan, Bis(4-aminobenzoxazolin-2-on-6-yl)-propan, Bis(4-aminobenzoxazolin-2-on-6-yl)-propan, Bis(5-aminobenzoxazolin-2-on-6-yl)-ether, Bis(7-aminobenzoxazolin-2-on-5-yl)-ether, Bis(6-aminobenzoxazolin-2-on-5-yl)-sulfid oder Bis(4-aminobenzoxazolin-2-on-5-yl)-sulfon.

Anstelle dieser beispielhaft genannten primären Aminoverbindungen können beim erfindungsgemäßen Verfahren selbstverständlich auch die entsprechenden sekundären Amine ($R^1 = C_1 - C_4$-Alkyl) eingesetzt werden, obwohl die Verwendung von primären Aminen beim erfindungsgemäßen Verfahren bevorzugt ist.

Die Herstellung der erfindungswesentlichen Verbindungen der genannten allgemeinen Formel, für welche $R^2$ und $R^3$ für Wasserstoff, einen $C_1 - C_4$-Alkylrest oder Chlor stehen, kann beispielsweise nach der von J. Sam und J.L. Valentine in J. Pharm. Sci. 58, 1043 ff (1969) beschriebenen Methode erfolgen. Die Herstellung der erfindungswesentlichen Verbindungen der genannten allgemeinen Formel, für welche $R^2$ und $R^3$ für einen ankondensierten, gegebenenfalls aminosubstituierten Benzolring stehen, erfolgt beispielsweise dergestalt, daß man o-Aminonaphthole mit Phosgen oder Harnstoff in ein einen ankondensierten Oxazolin-2-on-Ring aufweisendes Naphthalin überführt, welches durch Nitrierung und anschließende Reduzierung der Nitrogruppe in die erfindungswesentliche Verbindung überführt werden kann. Die Herstellung von erfindungswesentlichen Verbindungen der genannten allgemeinen Formel, für welche $R^3$ für einen Rest der Formel

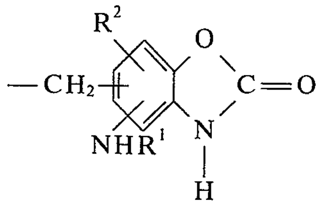

steht, kann beispielsweise nach folgendem Formelschema durch an sich bekannte Umsetzungen erfolgen:

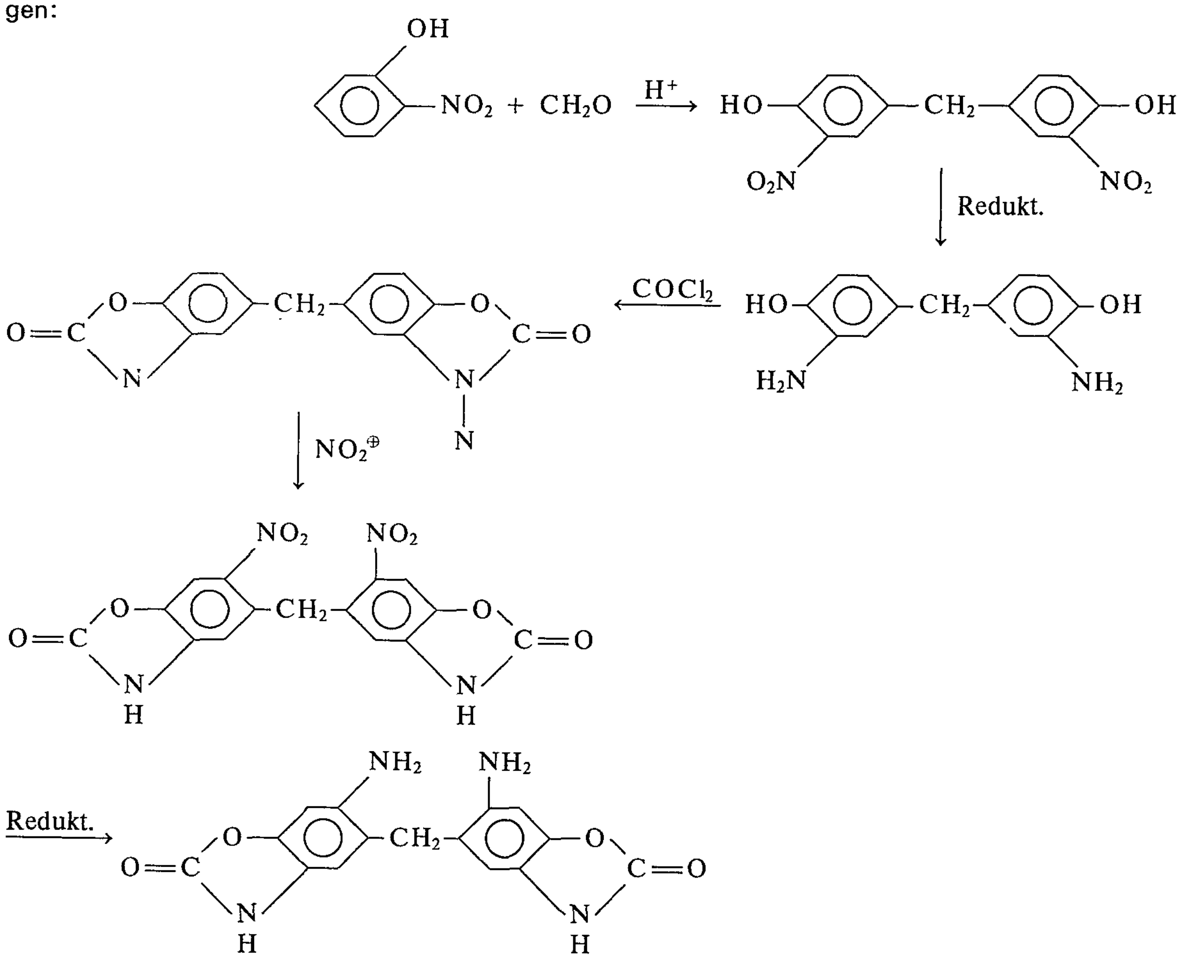

Reaktionspartner für die erfindungswesentlichen Verbindungen der allgemeinen Formel, welche mindestens eine aromatisch gebundene Aminogruppe und mindestens einen ankondensierten Oxazolin-2-on-Ring aufweisen, sind beliebige organische Verbindungen, welche mindestens zwei Gruppierungen aufweisen, welche mit primären oder sekundären aromatischen Aminogruppen im Sinne einer Additions- oder Kondensationsreaktion zu reagieren vermögen. Geeignete derartige Verbindungen sind

1) organische Polyisocyanate, wie z. B. aliphatische, cycloaliphatische, araliphatische, aromatische und heterocyclische Polyisocyanate, wie sie z. B. von W. Siefken in Justus Liebigs Annalen der Chemie, 562, Seiten 75 bis 136, beschrieben werden, beispielsweise solche der Formel

$Q(NCO)_n$

in der

n =2–4, vorzugsweise 2, und
Q einen aliphatischen Kohlenwasserstoffrest mit 2–18, vorzugsweise 6–10 C-Atomen, einen cycloaliphatischen Kohlenwasserstoffrest mit 4–15, vorzugsweise 5–10 C-Atomen, einen aromatischen Kohlenwasserstoffrest mit 6–15, vorzugsweise 6–13 C-Atomen, oder einen araliphatischen Kohlenwasserstoffrest mit 8–15, vorzugsweise 8–13 C-Atomen,

bedeuten, z. B. 1,6-Hexamethylendiisocyanat, 1,12-Dodecandiisocyanat, Cyclobutan-1,3-diisocyanat, Cyclohexan-1,3- und -1,4-diisocyanat sowie beliebige Gemische dieser Isomeren, 1-Isocyanato-3,3,5-trimethyl-5-isocyanatomethyl-cyclohexan (DE-Auslegeschrift 1 202 785, US-Patentschrift 3 401 190), 2,4- und 2,6-Hexahydrotoluylendiisocyanat sowie beliebige Gemische dieser Isomeren, Perhydro-2,4'- und/oder -4,4-diphenylmethan-diisocyanat, 1,3- und 1,4-Phenylendiisocyanat, 2,4- und 2,6-Toluylendiisocyanat sowie beliebige Gemische dieser Isomeren, Diphenylmethan-2,4'- und/oder -4,4'-diisocyanat, Naphthylen-1,5-diisocyanat. Ferner kommen beispielsweise erfindungsgemäß in Frage: Polyphenyl-polymethylenpolyisocyanate, wie sie durch Anilin-Formaldehyd-kondensation und anschließende Phosgenierung erhalten und z. B. in den GB-Patentschriften 874 430 und 848 671 beschrieben werden, Allophanatgruppen aufweisende Polyisocyanate, wie sie z. B. in der GB-Patentschrift 994 890, der BE-Patentschrift 761 626 und der NL-Patentanmeldung 7 102 524 beschrieben werden, Isocyanuratgruppen aufweisende Polyisocyanate, wie sie z. B. in der US-Patentschrift 3 001 973, in den DE-Patentschriften 1 022 789, 1 222 067 und 1 027 394 sowie in den DE-Offenlegungsschriften 1 929 034 und 2 004 048 beschrieben werden.

Für das erfindungsgemäße Verfahren besonders bevorzugte organische Polyisocyanate sind freie Isocyanatgruppen aufweisende Prepolymere, wie sie in an sich bekannter Weise durch Umsetzung von einfach organischen Polyisocyanaten der beispielhaft genannten Art mit unterschüssigen Mengen an organischen Polyhydroxylverbindungen des Molekulargewichtsbereichs 400 bis 10 000 erhalten werden. Diese NCO-Prepolymeren weisen im allgemeinen einen NCO-Gehalt von 0,5 bis 26 vorzugsweise 1 bis 15 Gew.-% auf.

Zur Herstellung dieser NCO-Präpolymeren geeignete mehrwertige Alkohole sind insbesondere die in der Polyurethanchemie an sich bekannten Polyether- oder Polyesterpolyole des genannten Molekulargewichtsbereichs, insbesondere solche mit einem zwischen 500 und 8000, vorzugsweise 1000 bis 6000 liegenden Molekulargewichts, die in der Regel 2 bis 8, vorzugsweise 2 bis 4 und besonders bevorzugt 2 alkoholische Hydroxylgruppen aufweisen.

Die in Frage kommenden Hydroxylgruppen aufweisenden Polyester sind z. B. Umsetzungsprodukte von mehrwertigen, vorzugsweise zweiwertigen und gegebenenfalls zusätzlich dreiwertigen Alkoholen mit mehrwertigen, vorzugsweise zweiwertigen, Carbonsäuren. Anstelle der freien Polycarbonsäuren können auch die entsprechenden Polycarbonsäureanhydride oder entsprechende Polycarbonsäureester von niedrigen Alkoholen oder deren Gemische zur Herstellung der Polyester verwendet werden. Die Polycarbonsäuren können aliphatischer, cycloaliphatischer, aromatischer und/oder heterocyclischer Natur sein und gegebenenfalls, z. B. durch Halogenatome, substituiert und/oder ungesättigt sein.

Als Beispiele für solche Carbonsäuren und deren Derivate seien genannt:

Bernsteinsäure, Adipinsäure, Korksäure, Azelainsäure, Sebacinsäure, Phthalsäure, Isophthalsäure, Trimellitsäure, Phthalsäureanhydrid, Tetrahydrophthalsäureanhydrid, Hexahydrophthalsäureanhydrid, Tetrachlorphthalsäureanhydrid, Endomethylentetrahydrophthalsäureanhydrid, Glutarsäureanhydrid, Maleinsäure, Maleinsäureanhydrid, Fumarsäure, dimerisierte und trimerisierte ungesättigte Fettsäuren, gegebenenfalls in Mischung mit Monomeren ungesättigten Fettsäuren, wie Ölsäure; Terephthalsäuredimethylester und Terephthalsäure-bis-glykolester. Als mehrwertige Alkohole kommen z. B. Ethylenglykol, Propylenglykol-(1,2) und -(1,3), Butylenglykol-(1,4) und -(2,3), Hexandiol-(1,6), Octandiol-(1,8), Neopentylglykol, 1,4-Bis-hydroxymethylcyclohexan, 2-Methyl-1,3-propandiol, Glycerin, Trimethylolpropan, Hexantriol-(1,2,6), Butantriol-(1,2,6), Trimethylolethan, Pentaerythrit, Chinit, Mannit und Sorbit, Formit, Methylglykosid, ferner Diethylenglykol, Triethylenglykol, Tetraethylenglykol und höhere Polyethylenglykole, Dipropylenglykol und höhere Polypropylenglykole sowie Dibutylenglykol und höhere Polybutylenglyklole in Frage. Die Polyester können anteilig endständige Carboxylgruppen aufweisen. Auch Polyester aus Lactonen, z. B. ε-Caprolacton, oder aus Hydroxycarbonsäuren, z. B. ω-Hydroxycapronsäure, sind einsetzbar.

Zur Herstellung der NCO-Prepolymeren geeignete Polyetherpolyole sind ebenfalls solche der an sich bekannten Art. Sie werden z. B. durch Polymerisation von Epoxiden wie Ethylenoxid, Propylenoxid, Butylenoxid, Tetrahydrofuran, Styroloxid oder Epichlorhydrin mit sich selbst, z. B. in Gegenwart von Lewis-Katalysatoren wie $BF_3$, oder durch Anlagerung dieser Epoxide, vorzugsweise von Ethylenoxid und Propylenoxid, gegebenenfalls im Gemisch oder nacheinander, an Startkomponenten mit reaktionsfähigen Wasserstoffatomen wie Wasser, Alkohole, Ammoniak oder Amine, z. B. Ethylenglykol, Propylenglykol-(1,3) oder -(1,2), Trimethylolpropan, Glycerin, Sorbit, 4,4'-Dihydroxy-diphenylpropan, Anilin, Ethanolamin oder Ethylendiamin hergestellt. Auch Sucrosepolyether, wie sie z. B. in den DE-Auslegeschriften 1 176 358 und 1 064 938 beschrieben werden, sowie auf Formit oder Formose gestartete Polyether (DE-Offenlegungsschriften 2 639 083 bzw. 2 737 951), kommen erfindungsgemäß in Frage. Vielfach sind solche Polyether bevorzugt, die überwiegend (bis zu 90 Gew.-%, bezogen auf alle vorhandenen OH-Gruppen im Polyether) primäre OH-Gruppen aufweisen.

Typische Beispiele geeigneter Polyester oder Polyetherpolyole sind auch in High Polymers, Vol. XVI, »Polyurethanes, Chemistry and Technology«, verfaßt von Saunders-Frisch, Interscience Publishers, New York, London, Band I, 1962, Seiten 32—42 und Seiten 44—54 und Band II, 1964, Seiten 5—6 und 198—199, sowie im Kunststoff-Handbuch, Band VII, Vieweg-Höchtlen, Carl-Hanser-Verlag, München, 1966, z. B. auf den Seiten 45—71, beschrieben. Selbstverständlich können Mischungen der obengenannten Verbindungen mit mindestens zwei gegenüber Isocyanaten reaktionsfähigen Wasserstoffatomen mit einem Molekulargewicht von 400—10 000, z. B. Mischungen von Polyethern und Polyestern, eingesetzt werden.

Bei der Herstellung der für das erfindungsgemäße Verfahren geeigneten NCO-Prepolymeren können anstelle oder zusätzlich zu den genannten höhermolekularen Polyhydroxylverbindungen selbstverständlich auch die aus der Polyurethanchemie bekannten, im allgemeinen jedoch weniger bevorzugten Polythioether, Polyacetale, Polycarbonate oder Polyesteramide mit endständigen Hydroxylgruppen oder auch bereits Urethan- oder Harnstoffgruppen enthaltende Polyhydroxylverbindungen eingesetzt werden. Außerdem ist selbstverständlich bei der Herstellung der NCO-Prepolymeren die Mitverwendung von einfachen niedermolekularen Polyolen des Molekulargewichtsbereichs 62—400 wie z. B. von Ethylenglykol, Neopentylglykol, Hexamethylenglykol, Glycerin oder Trimethylolpropan möglich. Auch die aus der Polyurethanchemie an sich bekannten Diamine wie z. B. Hexamethylendiamin oder Isophorondiamin können bei der Herstellung der NCO-Prepolymeren in unterschüssigen Mengen mitverwendet werden, obwohl der Einsatz derartiger Verbindungen weniger bevorzugt ist.

2) Polyepoxide wie z. B. Glycidylether, die durch Epoxidierung der entsprechenden Allylether oder nach bekannten Verfahren durch Umsetzung eines molaren Überschusses von Epichlorhydrin mit einer aromatischen Polyhydroxyverbindung, wie z. B. Isopropylidenbisphenol, einem Novolak, Resorcin oder ähnlichen hergestellt werden. Besonders bevorzugt sind die Epoxidderivate von Methylen- oder Isopropylidenbisphenolen. In den nachfolgenden Beispielen wurde als Epoxidharz der Diglycidylether von 4,4'-Isopropylidenbisphenol eingesetzt.

Eine verbreitet angewandte Gruppe von gemäß der Erfindung geeigneten Polyepoxiden sind die harzartigen Epoxidpolyether, die durch Umsetzung eines Epihalohydrins, wie Epichlorhydrin, mit einem Polyhydroxyphenol oder einem Polyhydroxyalkohol erhalten werden. Die Epoxidharze haben durchschnittlich mindestens 2 reaktive 1,2-Epoxidgruppen pro Molekül. Beispiele für geeignete Dihydroxyphenole sind: 4,4'-Isopropylidenbisphenol, 2,4'-Dihydroxydiphenylethylmethan, 3,3'-Dihydroxydiphenyldiethylmethan, 3,4'-Dihydroxydiphenylmethylpropylmethan, 2,3'-Dihydroxydiphenylethylphenylmethan, 4,4'-Dihydroxydiphenylpropylphenylmethan, 4,4'-Dihydroxydiphenylbutylphenylmethan, 2,2'-Dihydroxydiphenylditolylmethan, 4,4'-Dihydroxydiphenyltolylmethan und ähnliche. Andere Polyhydroxyphenole, die ebenfalls mit einem Epihalohydrin zur Herstellung dieser Epoxidpolyether umgesetzt werden können, sind Verbindungen, wie Resorcin, Hydrochinon, substituierte Hydrochinone, wie z. B. Methylhydrochinon und ähnliche.

Unter den Polyhydroxyalkoholen, die mit einem Epihalohydrin zur Herstellung dieser harzartigen Epoxidpolyether umgesetzt werden können, sind Verbindungen wie Ethylenglykol, Propylenglykole, Butylenglykole, Pentandiole, Bis-(4-hydroxycyclohexyl)-dimethylmethan, 1,4-Dimethylolbenzol, Glycerin, 1,2,6-Hexantriol, Trimethylolpropan, Mannit, Sorbit, Erythrit, Pentaerythrit, Dimere, Trimere und höhere Polymere derselben, wie z. B. Polyethylenglykole, Polypropylenglykole, Triglycerin oder Dipentaerythrit, Polyallylalkohol, Polyhydroxythioether, wie z. B. 2,2', 3,3'-Tetrahydroxydipropylsulfid, Mercaptoalkohole wie Monothioglycerin oder Dithioglycerin, partiell veresterte Polyhydroxyalkohole wie Monostearin oder Pentaerythritmonoacetat und halogenierte Polyhydroxyalkohole wie die Monochlorhydrine von Glycerin, Sorbit oder Pentaerythrit.

Eine andere Gruppe von polymeren Polyepoxiden, die gemäß der Erfindung angewendet und durch Amin gehärtet werden können, sind die Epoxid-Novolak-Harze, die durch Umsetzung eines Epihalohydrins, wie Epichlorhydrin, mit dem harzartigen Kondensat eines Aldehyds, z. B. Formaldehyd mit entweder einem Monohydroxyphenol, wie z. B. Phenol selbst oder einem Polyhydroxyphenol

vorzugsweise in Gegenwart eines basischen Katalysators, wie z. B. Natrium- oder Kaliumhydroxid, erhalten werden. Weitere Einzelheiten über die Eigenschaften und Herstellung dieser Epoxid-Novolak-Harze können aus H. Lee und K. Neville, Handbook of Epoxy Resins, McGraw Hill Book Co., New York, 1967, entnommen werden.

3) Carbonsäurechloride, Ester oder Carbonsäureanhydride mit mindestens zwei Säurechlorid-, Ester- oder Anhydridgruppen, wie z. B. Glutarsäuredichlorid Adipinsäuredichlorid, Pimelinsäuredichlorid, Korksäuredichlorid, Azelainsäuredichlorid, Sebazinsäuredichlorid, Isophthalsäuredichlorid, Terephthalsäuredichlorid, Glutarsäuredimethyleseter, Adipinsäuredimethylester, Pimelinsäuredimethylester, Adipinsäurediethylester, Korksäuredimethylester, Azelainsäuredimethylester, Sebazinsäuredimethylester, Isophthalsäuredimethylester, Terephthalsäuredimethylester.

1,2,4,5-Benzoltetracarbonsäuredianhydrid, Bicyclo/2,2,2/octen-(7)-2,3 : 5,6-tetracarbonsäureanhydrid, Perylen-3,4 : 9,10-tetracarbonsäuredianhydrid oder Benzophenon-3,4,3',4'-tetracarbonsäuredianhydrid.

Bei der Durchführung des erfindungsgemäßen Verfahrens kommen die Reaktionspartner in solchen Mengen zum Einsatz, daß auf jede primäre bzw. sekundäre Aminogruppe des aromatischen Amins mit ankondensiertem Oxazolin-2-on-Ring 0,95 bis 5 Reaktivgruppen zur Verfügung stehen, die mit den Aminogruppen im Sinne einer Additions- oder Kondensationsreaktion zu reagieren vermögen. Bei der Verwendung von organischen Polyisocyanaten liegt das Äquivalentverhältnis zwischen Isocyanatgruppen und Aminogruppen vorzugsweise innerhalb des Bereichs von 0,95 : 1 und 2,5 : 1. Bei Verwendung der Reaktionspartner in Mengenverhältnissen, die einem Äquivalentverhältnis zwischen Isocyanatgruppen und Aminogruppen von 0,95 : 1 bis 1,5 : 1, insbesondere 1 : 1 bis 1,2 : 1 entsprechen, entstehen beim erfindungsgemäßen Verfahren in erster Linie NCO-Gruppen-freie Umsetzungsprodukte (die überschüssigen NCO-Gruppen werden im allgemeinen durch Nebenreaktionen verbraucht), welche in Abhängigkeit von der Funktionalität des Polyisocyanats und dem Gehalt des aromatischen Amins an ankondensierten Oxazolin-2-on-Ringen 2 bis 6 derartige Oxazolin-2-on-Ringe aufweisen. Diese Verfahrensprodukte sind im allgemeinen nicht selbstvernetzend, können jedoch unter Mitverwendung geeigneter Kettenverlängerungs- oder Vernetzungsmittel der nachstehend beispielhaft genannten Art in hochmolekulare Kunststoffe überführt werden.

Bei Verwendung der organischen Polyisocyanate in Mengen, die einem $NCO/NH_2$-Äquivalentverhältnis von mehr als 1,5 : 1, insbesondere von 2 : 1 bis 2,5 : 1, entsprechen, entstehen beim erfindungsgemäßen Verfahren Umsetzungsprodukte, welche neben dem ankondensierten Oxazolin-2-on-Ring noch freie Isocyanatgruppen aufweisen. Derartige Umsetzungsprodukte sind wegen der Reaktionsbereitschaft zwischen den Oxazolin-2-on- und Isocyanatgruppen in der Hitze selbstvernetzend.

Bei der Durchführung des erfindungsgemäßen Verfahrens unter Verwendung der unter 2) genannten Polyepoxide entstehen in völliger Analogie zu den soeben gemachten Ausführungen je nach Äquivalentverhältnis zwischen Epoxid- und Aminogruppen Umsetzungsprodukte, die neben ankondensierten Oxazolin-2-on-Ringe und, durch Ringöffnung der Epoxidgruppen, freie Hydroxylgruppen, sowie außerdem gegebenenfalls noch überschüssige Epoxidgruppen enthalten. Auch hier ist die Frage der Selbstvernetzbarkeit der Umsetzungsprodukte vom Gehalt an überschüssigen Epoxidgruppen abhängig.

Bei der Durchführung des erfindungsgemäßen Verfahrens unter Verwendung der unter 3) genannten Säurederivate wird vorzugsweise in einem Äquivalentverhältnis zwischen gegenüber Aminogruppen reaktionsfähiger Gruppe und Aminogruppe von 0,95 : 1 bis 1,1 : 1 gearbeitet, so daß als Umsetzungsprodukte keine selbstvernetzenden Verbindungen entstehen, die jedoch wegen ihres Gehalts an Oxazolin-2-on-Ringen unter Verwendung der nachstehend beispielhaft genannten Kettenverlängerungs- bzw. Vernetzungsmittel kettenverlängert bzw. vernetzt werden können.

Bei der Durchführung des erfindungsgemäßen Verfahrens richtet sich die Reaktionstemperatur nach der Art des Reaktionspartners für die aromatischen Amine mit ankondensiertem Oxazolin-2-on-Ring. Im Falle der Verwendung von organischen Polyisocyanaten wird das erfindungsgemäße Verfahren vorzugsweise im Temperaturbereich zwischen 20 und 100° C, insbesondere zwischen 50 und 90° C durchgeführt. Im Falle der Verwendung von Polyepoxiden liegt die Reaktionstemperatur im allgemeinen zwischen 40 und 120° C, insbesondere zwischen 70 und 100° C. Im Falle der Verwendung von Säurechloriden bzw. -anhydriden liegt die Reaktionstemperatur im allgemeinen zwischen 10 und 150° C, insbesondere zwischen 40 und 120° C. Im Falle der Verwendung von Carbonsäureestern liegt die Reaktionstemperatur vorzugsweise zwischen 80 und 200° C, insbesondere zwischen 110 und 160° C.

Das erfindungsgemäße Verfahren kann in Substanz oder in Anwesenheit geeigneter inerter Lösungsmittel durchgeführt werden. Beispiele geeigneter Lösungsmittel sind Dioxan, Tetrahydrofuran, Benzol, Toluol, Chlorbenzol, Nitrobenzol, Ethylenglykolmethylether, Diethylenglykoldimethylether, Ethylenglykolmonomethylethermonoacetat.

Wie bereits ausgeführt, entstehen beim erfindungsgemäßen Verfahren Umsetzungsprodukte, welche aromatische Ringsysteme mit ankondensierten Oxazolin-2-on-Ringen enthalten, wobei diese Ringsysteme mit den organischen Resten der oben unter 1)—3) genannten Reaktionspartner über Harnstoffgruppierungen (im Falle der Verwendung von Polyisocyanaten), sekundären oder tertiären

Aminogruppen (im Falle der Verwendung von Polyepoxiden) oder Amidgruppen (im Falle der Verwendung von Säurederivaten) verknüpft sind. Die beim erfindungsgemäßen Verfahren ablaufenden Additions- bzw. Kondensationsreaktionen können durch folgende Gleichungen verdeutlicht werden:

a) Reaktionen mit organischen Polyisocyanaten gemäß

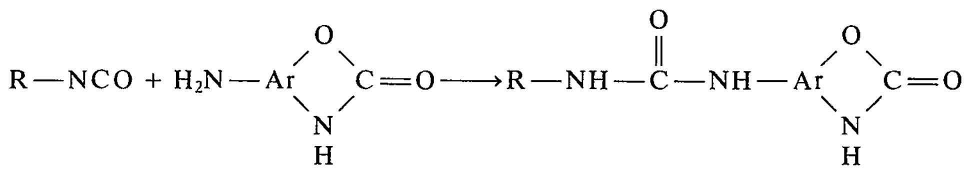

b) Reaktionen mit Polyepoxiden:

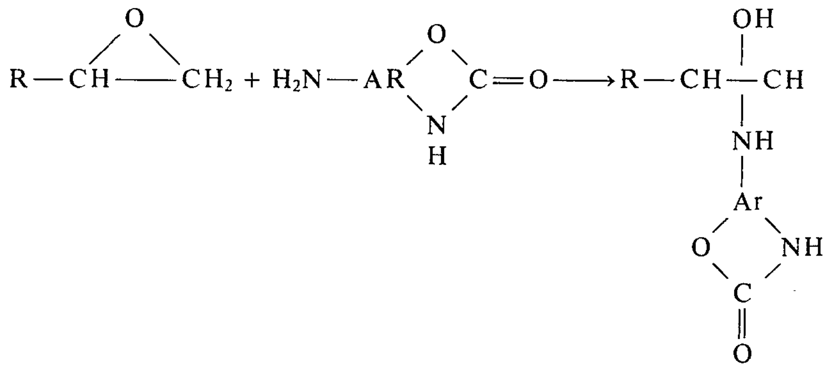

c) Reaktionen mit Säurechloriden oder Estern:

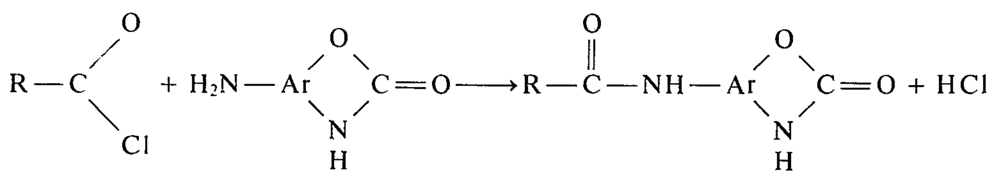

d) Reaktionen mit Carbonsäureanhydriden:

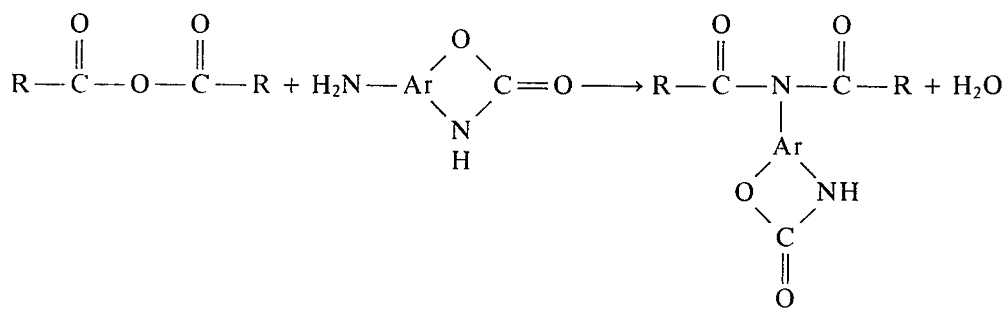

Die in den erfindungsgemäßen Verfahrensprodukten vorliegenden ankondensierten Oxazolin-2-on-Ringe sind nun, wie bereits ausgeführt, weiteren Reaktionen zugänglich. So reagiert die am Stickstoff des Oxazolin-2-on-Ringes gebundene Wasserstoffatom mit organischen Isocyanaten gemäß

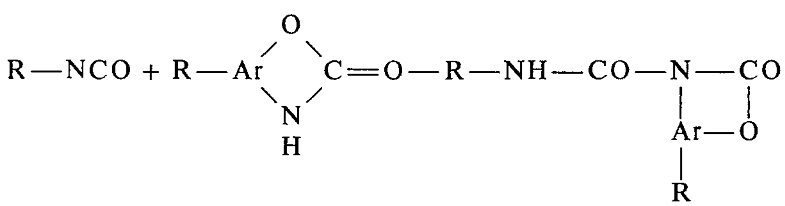

oder mit Epoxiden gemäß

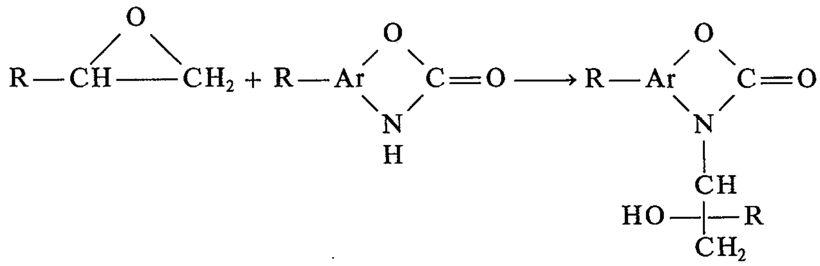

Mit primären oder sekundären Aminen reagieren die Oxazolin-2-on-Ringe gemäß folgender Formelschema:

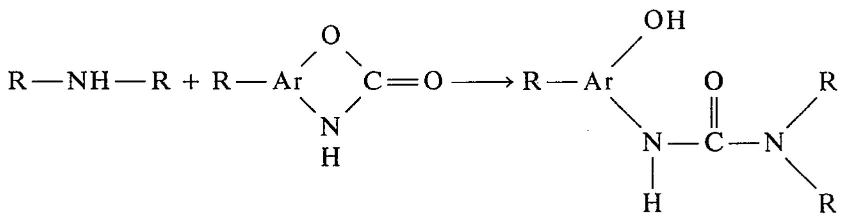

In diesen, lediglich zur Verdeutlichung der einzelnen Reaktionsmechanismen dienenden Gleichungen stehen die Reste R und Ar für die, bezüglich der Umsetzungen indifferenten, Reste der jeweiligen Reaktionspartner.

Diesen Ausführungen entsprechend können die erfindungsgemäßen Verfahrensprodukte beispielsweise mit organischen Polyisocyanaten, Polyepoxiden, mindestens difunktionellen Säurechloriden, Bis-Säureanhydriden oder organischen Polyaminen mit primären oder sekundären Aminogruppen vernetzt bzw. kettenverlängert werden. Falls in den erfindungsgemäßen Verfahrensprodukten noch freie Isocyanat- bzw. Epoxidgruppierungen vorliegen, stellen diese Verfahrensprodukte unter dem Einfluß von Hitze selbstvernetzende bzw. »selbstkettenverlängernde« Verbindungen dar. Grundsätzlich ist es auch möglich derartigen selbstvernetzenden oder »selbstkettenverlängernden« Verbindungen vor ihrer Vernetzung bzw. Kettenverlängerung ein Vernetzungs- bzw. Kettenverlängerungsmittel der nachstehend beispielhaft genannten Art zuzusetzen, so daß der Vernetzungseffekt verstärkt wird. Dies empfiehlt sich insbesondere oft im Falle der unter Verwendung von Polyepoxiden hergestellten Verfahrensprodukte, da in diesen außer den Oxazolin-2-on-Ringen auch noch durch die Ringöffnung der Epoxidgruppen entstandene Hydroxylgruppen vorliegen, die ebenfalls gegenüber Isocyanatgruppen oder Epoxidgruppen reaktionsfähig sein können.

Als Vernetzungs- bzw. Kettenverlängerungsmittel eignen sich

1. die oben beispielhaft genannten organischen Polyisocyanate, insbesondere die oben beispielhaft genannten NCO-Präpolymeren,
2. die oben beispielhaft genannten polyepoxide,
3. die oben beispielhaft genannten Säurederivate und
4. organische Polyamine, d. h. insbesondere aliphatische diprimäre Diamine mit 2 bis 12 Kohlenstoffatomen, diprimäre cycloaliphatische Diamine mit 6 bis 15 Kohlenstoffatomen, diprimäre aromatische Diamine mit 6 bis 15 Kohlenstoffatomen wie z. B. Ethylendiamin, Tetramethylendiamin, Hexamethylendiamin, Undecamethylendiamin, Dodecamethylendiamin, 1-Amino-3,3,5-trimethyl-5-aminomethylcyclohexan, 2,4- und 2,6-Hexahydrotoluylendiamin sowie deren Gemische, Perhydro-2,4'- und 4,4'-diaminodiphenylmethan, p-Xylylendiamin, Bis-(3-aminopropyl)-methylamin, 4,4'-Diaminodicyclohexylmethan, 3,3'-Dimethyl-4,4'-diaminodicyclohexylmethan, 3,5,3',5'-Tetraethyl-4,4'-diaminodicyclohexylmethan oder 2,2'-Bis-(4-aminocyclohexyl)-propan. Organische Polyamine, die beispielsweise durch Hydrolyse der NCO-Gruppen der oben beispielhaft genannten NCO-Präpolymeren erhalten werden können, sind ebenfalls als erfindungsgemäße Kettenverlängerungs- bzw. Vernetzungsmittel geeignet. Außerdem können cycloaliphatische Triamine beispielsweise solche gemäß DE-OS 2 614 244 oder sekundäre Amine wie z. B. Piperazin oder N,N',N''-Trimethyldiethylentriamin können als erfindungsgemäß verwendbare Vernetzer bzw. Kettenverlängerungsmittel eingesetzt werden.

Außer den oben beispielhaft genannten Kettenverlängerungs- bzw. Vernetzungsmitteln eignen sich auch Hydrazin oder Hydrazinderivate für diese Verwendung. Beispiele geeigneter Hydrazinderivate sind Methylhydrazin, N,N'-Dimethylhydrazin und deren Homologe, Säuredihydrazide wie z. B. Carbodihydrazid, Oxalsäuredihydrazid, die Dihydrazide von Malonsäure, Bernsteinsäure, Glutarsäure, Adipinsäure oder Sebacinsäuresemicarbazido-alkylen-hydrazide wie z. B. $\beta$-Semicarbazido-propion-

säurehydrazid (DE-OS 1 770 591 oder Semicarbazido-alkylencarbazinester wie z. B. 2-Semicarbazidoethyl-carbazinester (DE-OS 1 918 504).

Bei der erfindungsgemäßen Verwendung der erfindungsgemäßen Verfahrensprodukte werden die Vernetzungs- bzw. Kettenverlängerungsmittel im allgemeinen in solchen Mengen eingesetzt, daß auf jede gegenüber dem Vernetzungsmittel reaktionsfähige Gruppe der Verfahrensprodukte (der Oxazolin-2-on-Ring stellt hierbei eine monofunktionelle Gruppe dar), 0 bis 5, vorzugsweise 0,95 bis 1,05 reaktive Gruppen des Vernetzungs- bzw. Kettenverlängerungsmittels entfallen. Die Menge des Vernetzungsmittels kann, diesen Ausführungen entsprechend, je nach Gehalt der erfindungsgemäßen Verfahrensprodukte an Isocyanat- bzw. Epoxidgruppen verringert werden, so daß insbesondere bei Vorliegen äquivalenter Mengen an Isocyanatgruppen und Oxazolin-2-on-Ringen im Verfahrensprodukt auf den Einsatz der Vernetzungs- bzw. Kettenverlängerungsmittel völlig verzichtet werden kann.

Die Temperatur, bei welcher die Vernetzungs- bzw. Kettenverlängerungsreaktion abläuft hängt in erster Linie von der Natur des Vernetzungs- bzw. Kettenverlängerungsmittels ab. Sie liegt im Falle von organischen Polyisocyanaten, organischen Polyaminen, organischen Säureanhydriden oder organischen Säurechloriden im allgemeinen im Temperaturbereich zwischen 100 und 180° C, vorzugsweise 120 bis 160° C. Bei Verwendung von mindestens difunktionellen Carbonsäureestern oder von Polyepoxiden liegt die Vernetzungs- bzw. Kettenverlängerungstemperatur im allgemeinen innerhalb des Temperaturbereichs von 140 bis 200, vorzugsweise 150 bis 180° C.

Sowohl bei der Durchführung des erfindungsgemäßen Verfahrens als auch bei der erfindungsgemäßen Verwendung der erfindungsgemäßen Verfahrensprodukte können, die jeweilige Reaktion beschleunigende, Katalysatoren mitverwendet werden. So empfiehlt sich beispielsweise bei der Herstellung von Harnstoffgruppen aufweisenden erfindungsgemäßen Verfahrensprodukten unter Verwendung von organischen Polyisocyanaten als Ausgangsmaterialien oft der Einsatz der in der Polyurethanchemie üblichen tertiären Amine als Katalysatoren. Beispiele derartiger Katalysatoren sind Triethylendiamin oder N,N-Dimethylbenzylamin. Geeignete Katalysatoren für die Vernetzungs- bzw. Kettenverlängerungsreaktion unter Verwendung von organischen Polyaminen sind beispielsweise Titan-tetraalkylate wie z. B. Titan-tetrabutylat oder Titan-tetraethylat.

Bei dem Einsatz von organischen Säurechloriden bei der Durchführung des erfindungsgemäßen Verfahrens oder als Vernetzungs- bzw. Kettenverlängerungsmittel ist die Mitverwendung geeigneter Basen zur Neutralisation des sich bildenden Chlorwasserstoffs unerläßlich. Geeignete derartige Basen sind z. B. Triethylamin, Tripropylamin, Tributylamin, Pyridin, Piperidin, Morpholin, Piperazin.

Auch die Herstellung von hochmolekularen Kunststoffen unter erfindungsgemäßer Verwendung der erfindungsgemäßen Verfahrensprodukte kann in Gegenwart und in Abwesenheit geeigneter inerter Lösungsmittel der oben bereits beispielhaft genannten Art erfolgen.

Auch die Mitverwendung von Treibmitteln wie z. B. von in der Hitze gasabspaltenden Verbindungen wie Azocarbonamid ist möglich. In diesem Falle können bei der erfindungsgemäßen Verwendung geschäumte Kunststoffe erhalten werden. Falls als Vernetzungs- bzw. Kettenverlängerungsmittel organische Polyisocyanate eingesetzt werden, kann selbstverständlich auch Wasser als Treibmittel verwendet werden.

Durch geeignete Wahl der Ausgangsmaterialien bei der Durchführung des erfindungsgemäßen Verfahrens und auch durch geeignete Wahl der bei der erfindungsgemäßen Verwendung eingesetzten Vernetzungs- bzw. Kettenverlängerungsmittel können die mechanischen Eigenschaften der letztlich erhaltenen Kunststoffe wie beispielsweise deren Härte und Elastizität dem jeweiligen Verwendungszweck in optimaler Weise angepaßt werden. So kann beispielsweise die Elastizität der erfindungsgemäß erhältlichen Kunststoffe durch den Einbau langkettiger Polyester- bzw. Polyethersegmente erhöht werden. Die erfindungsgemäß erhältlichen Kunststoffe können zur Herstellung von massiven Formteilen, als Klebstoffe, als Schaumkunststoffe oder als Beschichtungen beliebiger Substrate Verwendung finden. Besonders hervorzuheben ist in letztgenanntem Zusammenhang, daß es sich bei den erfindungsgemäßen Verfahrensprodukten, wie beispielsweise aus ihrer Reaktionsbereitschaft mit Polyaminen ersichtlich, um blockierte Polyisocyanate handelt, die bei ihrer Verwendung keine flüchtigen Blockierungsmittel abspalten.

In den nachfolgenden Beispielen beziehen sich alle Prozentangaben auf Gewichtsprozent.

## Beispiel 1

50 g eines NCO-Prepolymeren, hergestellt durch Umsetzung von 2,4-Diisocyanatotoluol mit einem Polypropylenglykol des mittleren Molekulargewichts 2000, welches einen NCO-Gehalt von 3,4% aufweist, werden mit 3 g 5-Aminobenzoxazolin-2-on ($NCO/NH_2$-Äquivalentverhältnis = 2 : 1) gut vermischt und während 15 Minuten auf 80° C erhitzt. Es wird ein freie Isocyanatgruppen und Benzoxazolin-2-on-Ringe enthaltendes Umsetzungsprodukt erhalten, das durch 4stündiges Aushärten bei 140° C in einen elastischen Kunststoff überführt werden kann.

Beispiel 2

40,6 g eines Semiprepolymeren mit einem NCO-Gehalt von 13,7%, hergestellt durch Umsetzung von 1250 g 4,4'-Diisocyanatodiphenylmethan mit 56,5 g Butandiol-1,3 und 960 g eines trifunktionellen Polyetherpolyols des Molekulargewichts 4800, welches durch Propoxylierung von Trimethylolpropan erhalten worden war, werden mit 10g 4-Aminobenzoxazolin-2-on ($NCO/NH_2$-Äquivalentverhältnis = 2 : 1) vermischt und während 15 Minuten auf 70° C erhitzt. Es entsteht eine freie Isocyanatgruppen und Oxazolin-2-on-Ringe enthaltendes Umsetzungsprodukt, welches durch 9stündiges Aushärten bei 130° C in einen harten Kunststoff der Shore-Härte D 42 überführt werden kann.

Beispiel 3

45,2 g eines Semiprepolymeren mit einem NCO-Gehalt von 12,3%, hergestellt durch Umsetzung von 1250 g 4,4'-Diisocyanatodiphenylmethan mit 136 g Trimethylhexandiol-1,6 und 960 g des in Beispiel 2 genannten Polyetherpolyols, werden mit 10 g 4-Aminobenzoxazolin-2-on vermischt ($NCO/NH_2$-Äquivalentverhältnis = 2 : 1) und während 15 Minuten auf 70° C erhitzt. Es entsteht ein freie Isocyanatgruppen und Oxazolin-2-on-Ringe enthaltendes Umsetzungsprodukt, welches durch 2stündiges Erhitzen auf 130° C in einen harten Kunststoff der Shore-Härte D 45 überführt werden kann.

Beispiel 4

42 g eines Semiprepolymeren mit einem NCO-Gehalt von 13,3%, hergestellt durch Umsetzung von 1250 g 4,4'-Diisocyanatodiphenylmethan mit 88 g Neopentylglykol und 960 g des Polyetherpolyols aus Beispiel 2, werden mit 10 g 4-Aminobenzoxazolin-2-on ($NCO/NH_2$-Äquivalentverhältnis = 2 : 1) vermischt und während 15 Minuten auf 70°C erhitzt. Es entsteht ein freie Isocyanatgruppen und Oxazolin-2-on-Ringe enthaltendes Umsetzungsprodukt, welches durch 2stündiges Erhitzen auf 130° C in einen harten Kunststoff der Shore-Härte D 40 überführt werden kann.

Beispiel 5

16,8 g Hexamethylendiisocyanat werden mit 30 g 5-Aminobenzoxazolin-2-on ($NCO/NH_2$-Äquivalentverhältnis = 1 : 1) vermischt und während 20 Minuten auf 70°C erhitzt. Es entsteht ein Benzoxazolin-2-on-Ringe aufweisendes, isocyanatgruppenfreies Umsetzungsprodukt.

9,3 g dieses Umsetzungsprodukts werden mit 25 g eines organischen Diamins mit einem Gehalt an endständigen Aminogruppen von 2,6% in Gegenwart von 0,1 g Titantetrabutylat (Äquivalentverhältnis zwischen Oxazolin-2-on-Ringen und Aminogruppen = 1 : 1) vermischt und während 1 Stunde auf 140°C zu einem hochelastischen klebfreien Kunststoff ausgehärtet. Das hierbei eingesetzte Diamin war durch Hydrolyse der endständigen Isocyanatgruppen eines NCO-Prepolymeren auf Basis von Hexamethylendiisocyanat und Polypropylenglykol des Molekulargewichts 1000 hergestellt worden.

Beispiel 6

11,6 g 2,4-Diisocyanatotoluol werden mit 20 g 5-Aminobenzoxazolin-2-on ($NCO/NH_2$-Äquivalentverhältnis = 1 : 1) in Dioxan während 240 Minuten auf 100°C erhitzt. Es entsteht so ein bifunktionelles Oxazolin-2-on-Ringe enthaltendes, isocyanatgruppenfreies Umsetzungsprodukt, welches unter Verwendung des Diamins aus Beispiel 5 bei einem Äquivalentverhältnis der Reaktionspartner von 1 : 1 und unter gleichzeitiger Verwendung von Titantetrabutylat als Katalysator durch 4stündiges Erhitzen auf 140° C zu einem elastischen Kunststoff umgesetzt werden kann.

Beispiel 7

30 g 5-Aminobenzoxazolin-2-on (0,2 Mol) werden in 300 ml trockenem Dioxan gelöst und mit 20,2 g (0,2 Mol) Triethylamin versetzt. Zu dieser Lösung werden bei Raumtemperatur 18,3 g (0,1 Mol) Adipinsäuredichlorid innerhalb 30 Minuten zugetropft, wobei die Temperatur auf 40°C steigt. Anschließend wird während 2 Stunden bei 70° C nachgerührt und mit einem Liter Wasser versetzt, abgesaugt und mit weiterem Wasser chloridfrei gewaschen. Es resultieren 36 g (83%) eines grauen, oberhalb 300° C schmelzenden Produkts der Struktur

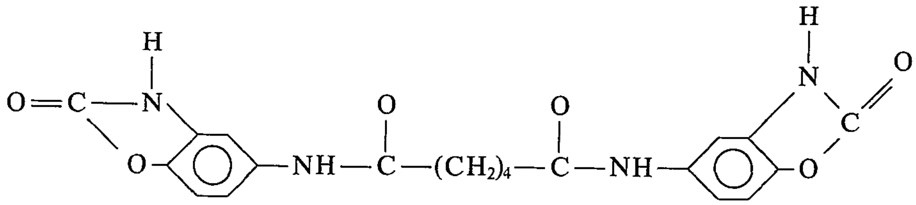

Analysen:

C (gefunden): 58,0%, berechnet: 58,5%;
H (gefunden): 4,4%, berechnet: 4,4%;
N (gefunden): 12,8%, berechnet: 13,6%.

Beispiel 8

30 g 6-Aminobenzoxazolin-2-on (0,2 Mol) werden in 200 ml trockenem Toluol suspendiert. Anschließend werden 0,5 g Triethylendiamin und 16,8 g 0,1 Mol Hexamethylendiisocyanat hinzugegeben. Nach 4stündigem Erhitzen auf 100° C war kein freies Isocyanat mehr nachzuweisen. Die unlösliche Festsubstanz wurde abgesaugt und mit je 200 ml Toluol und Petrolether gewaschen. Es wurden 46 g (98,3% der Theorie) einer graubraunen, oberhalb 260° C schmelzenden Verbindung folgender Struktur erhalten:

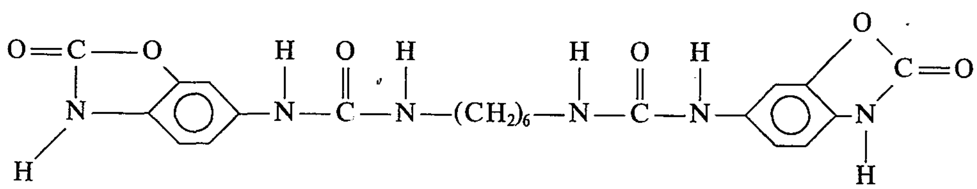

C (gefunden): 56,9%, berechnet: 56,4%;
H (gefunden): 5,4%, berechnet: 5,2%;
N (gefunden): 16,9%, berechnet: 17,9%.

Beispiel 9

20 g (0,133 Mol) 6-Aminobenzoxazolin-2-on wurden in 250 ml trockenem Dioxan gelöst und mit 11,6 g 2,4-Diisocyanatotoluol (0,0665 Mol) versetzt und 4 Stunden bei 100° C gehalten. Danach war kein freies Isocyanat mehr nachweisbar. Die unlösliche Festsubstanz wurde abgesaugt und mit 200 ml Petrolether gewaschen. Es werden 30 g (95%) einer graubraunen, in Dimethylformamid löslichen, über 300° C schmelzenden Verbindung der folgenden Struktur erhalten:

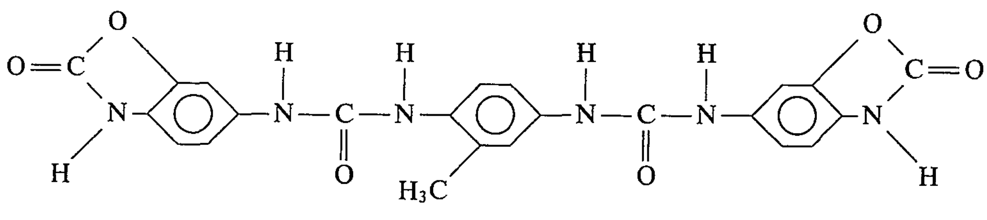

C (gefunden): 58,6%, berechnet: 59,1%;
H (gefunden): 3,8%, berechnet: 3,7%;
N (gefunden): 16,8%, berechnet: 17,3%.

Beispiel 10

34,8 g (0,199 Mol) 2,4-Diisocyanatotoluol werden in 250 ml trockenem Dioxan gelöst. Zu dieser Lösung werden bei 80° C während 60 Minuten 30 g 6-Aminobenzoxazolin-2-on (0,199 Mol) portionsweise zugegeben. Nach 4stündigem Erhitzen auf 80° C werden 50 g (59% der Theorie) eines schwer löslichen graubraunen Feststoffs mit einem NCO-Gehalt von 12,4% erhalten.

**Patentansprüche**

1. Verfahren zur Herstellung von unter dem Einfluß von Hitze und gegebenenfalls in Gegenwart von Kettenverlängerungs- bzw. Vernetzungsmitteln zu hochmolekularen, gegebenenfalls vernetzten Kunststoffen ausreagierenden, Oxazolin-2-on-Ringe aufweisenden Kunststoffvorläufern, dadurch gekennzeichnet, daß man mindestens zwei mit primären oder sekundären, aromatisch gebundenen Aminogruppen im Sinne einer Additions- oder Kondensationsreaktion reaktionsfähige Gruppierungen aufweisende Kunststoffvorläufer, ausgewählt aus der Gruppe bestehend aus organischen Polyisocyanaten, Polyepoxiden, Carbonsäurechloriden mit mindestens zwei Säurechloridgruppen, Carbonsäureestern mit mindestens zwei Estergruppen und Carbonsäureanhydriden mit mindestens zwei Anhydridgruppen, mit mindestens einen ankondensierten Oxazolin-2-on-Ring aufweisenden aromatischen Aminen mit mindestens einer primären oder sekundären aromatisch gebundenen Aminogruppe der allgemeinen Formel

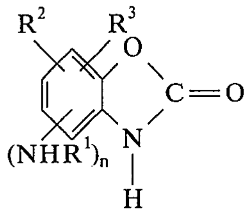

in welcher

$R^1$ für Wasserstoff oder einen aliphatischen Kohlenwasserstoffrest mit 1–4 Kohlenstoffatomen steht,

$R^2$ und $R^3$ entweder für gleiche oder verschiedene Reste stehen und Wasserstoff, einen aliphatischen Kohlenwasserstoffrest mit 1–4 Kohlenstoffatomen oder Chlor bedeuten oder gemeinsam für einen ankondensierten, gegebenenfalls mit einer Aminogruppe $-NHR^1$ substituierten Benzolring stehen oder wobei $R^2$ die eingangs genannte Bedeutung hat und $R^3$ für einen Rest der Formel

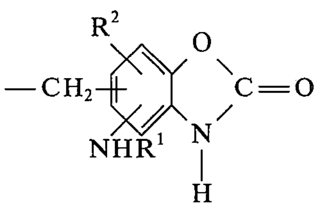

steht, und

n für 1 steht bzw. im Falle des Vorliegens eines aminosubstituierten, ankondensierten aromatischen Rings auch für 0 stehen kann, im Sinne einer Additions- oder Kondensationsreaktion umsetzt, wobei man die Mengen der Reaktionspartner so wählt, daß auf jede aromatisch gebundene primäre oder sekundäre Aminogruppe 0,95 bis 5 an mit diesen Aminogruppen reaktionsfähige Gruppen des Kunststoffvorläufers entfallen.

2. Verfahren gemäß Anspruch 1, dadurch gekennzeichnet, daß man als organische Polyisocyanate freie Isocyanatgruppen aufweisende Polyurethan-Präpolymere verwendet, welche Umsetzungsprodukte überschüssiger Mengen einfacher organischer Polyisocyanate mit organischen Polyhydroxylverbindungen des Molekulargewichtsbereichs 400 bis 10 000 darstellen.

3. Verfahren gemäß Anspruch 1 und 2, dadurch gekennzeichnet, daß man die Reaktionspartner in solchen Mengenverhältnissen miteinander umsetzt, daß auf jede Aminogruppe 0,95 bis 2,5 Isocyanatgruppen des organischen Polyisocyanats entfallen.

4. Verwendung der gemäß Anspruch 1 bis 3 erhältlichen, Oxazolin-2-on-Ringe aufweisenden Kunststoffvorläufer zur Herstellung von hochmolekularen Kunststoffen, dadurch gekennzeichnet, daß man die Kunststoffvorläufer gegebenenfalls in Gegenwart von Verbindungen, die mindestens zwei, mit Oxazolin-2-on-Ringen im Sinne einer Additions- oder Kondensationsreaktion reaktionsfähigen Gruppen einer Hitzebehandlung im Bereich von 100 bis 200° C unterwirft.

5. Verwendung gemäß Anspruch 4, dadurch gekennzeichnet, daß man als Verbindungen mit gegenüber Oxazolin-2-on-Ringen reaktionsfähigen Gruppen organische Polyisocyanate oder organische Polyamine mit primären und/oder sekundären Aminogruppen verwendet.

## Claims

1. Process for the preparation of polymer precursors containing oxazolin-2-one rings, which polymer precursors react under the influence of heat and the optional presence of chain-lengthening or cross-linking agentes to form high molecular weight, possibly cross-linked polymers, characterised in that polymer precursors which have at least two groups capable of entering into an addition or condensation reaction with primary or secondary, aromaticallybound amino groups and are selected from the group consisting of organic polyisocyanates, polyepoxides, carboxylic acid chlorides having at least two acid chloride groups, carboxylic acid esters having at least two ester groups and carboxylic acid anhydrides having at least two anhydride groups, are subjected to an addition or condensation reaction with aromatic amines having at least one primary or secondary, aromatically-bound amino group and containing at least one condensed oxazolin-2-one ring, of the general formula

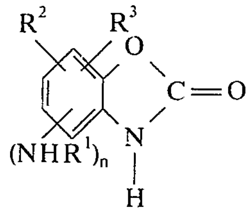

in which

$R^1$ represents hydrogen or an aliphatic hydrocarbon radical with 1—4 carbon atoms,

$R^2$ and $R^3$ either represent identical or different radicals and denote hydrogen, an aliphatic hydrocarbon radical with 1—4 carbon atoms or chlorine, or together represent a condensed benzene ring optionally substituted by an amino group $-NHR^1$, or wherein $R^2$ has the meaning given at the beginning and $R^3$ represents a radical of the formula

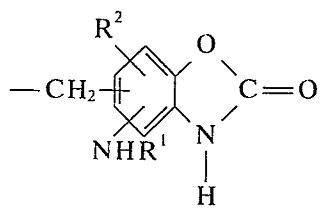

and

n represents 1, or if an amino-substituted, condensed aromatic ring is present, can also represent 0, the quantities of the reactants being selected such that for each aromatically-bound primary or secondary amino group 0.95 to 5 groups of the polymer precursor which are capable of reacting with these amino groups are present.

2. Process according to Claim 1, characterised in that the organic polyisocyanates used are polyurethane prepolymers containing free isocyanate groups, which prepolymers are reaction products of excess quantities of simple organic polyisocyanates with organic polyhydroxyl compounds having molecular weights in the range of 400 to 10,000.

3. Process according to Claim 1 and 2, characterised in that the reactants are reacted with one another in such proportions that there are 0.95 to 2.5 isocyanate groups of the organic polyisocyanate for each amino group.

4. Use of the polymer precursors containing oxazolin-2-one rings and obtainable according to Claim 1 to 3 for the production of high molecular weight polymers, characterised in that the polymer precursors are subjected to a heat treatment at a temperature in the range from 100 to 200°C, optionally in the presence of compounds which have at least two groups capable of undergoing an addition or condensation reaction with oxazolin-2-one rings.

5. Use according to Claim 4, characterised in that the compounds used containing groups which are reactive with oxazolin-2-one rings are organic polyisocyanates or organic polyamines having primary and/or secondary amino groups.

## Revendications

1. Procédé de production de progéniteurs de matières plastiques porteurs de noyaux d'oxazoline-2-one, réagissant sous l'influence de la chaleur et le cas échéant en présence d'agents

d'allongement de chaine ou de réticulation en formant des matières plastiques macromoléculaires éventuellement réticulées, caractérisé en ce qu'on fait réagir, au sens d'une réaction d'addition ou de condensation, des progéniteurs de matières plastiques portant au moins deux groupements aptes à réagir, au sens d'une réaction d'addition ou de condensation, avec des groupes amino primaires ou secondaires en liaison aromatique, choisis dans le groupe formé de polyisocyanates organiques, de polyépoxydes, de chlorures d'acides carboxyliques ayant au moins deux groupes chlorure d'acide, d'esters d'acides carboxyliques ayant au moins deux groupes ester et d'anhydrides d'acides carboxyliques ayant au moins deux groupes anhydride, avec des amines aromatiques portant au moins un noyau condensé d'oxazoline-2-one, et présentant au moins un groupe amino primaire ou secondaire en liaison aromatique, de formule générale

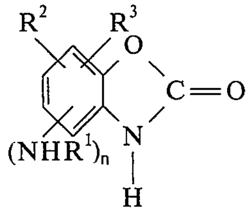

dans laquelle

$R^1$ représent l'hydrogène ou un reste d'hydrocarbure aliphatique ayant 1 à 4 atomes de carbone,
$R^2$ et $R^3$ représentent des restes égaux ou différents et l'hydrogène, un reste d'hydrocarbure aliphatique ayant 1 à 4 atomes de carbone ou le chlore ou forment en commun un noyau benzénique condensé, éventuellement substitué avec un groupe amino $-NHR^1$, ou bien $R^2$ a la définition donnée ci-dessus et $R^3$ est un reste de formule

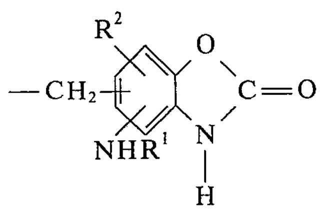

et

n est égal à 1 ou bien, dans le cas de la présence d'un noyau aromatique condensé à substituant amino, n peut aussi être égal à 0, et on choisit alors les quantités des partenaires réactionnels de manière qu'il y ait, pour chaque groupe amino primaire ou secondaire en liaison aromatique, 0,95 à 5 groupes, réactifs avec ces groupes amino, du progéniteur de matières plastiques.

2. Procédé suivant la revendication 1, caractérisé en ce qu'on utilise comme polyisocyanates organiques des prépolymères de polyuréthanes porteurs de groupes isocyanate libres, qui représentent des produits de réaction de quantitités en excès de polyisocyanates organiques simples avec des composés polyhydroxyliques organiques de poids moléculaire compris dans l'intervalle de 400 à 10 000.

3. Procédé suivant les revendications 1 et 2, caractérisé en ce qu'on fait réagir ensemble les partenaires réactionnels dans des rapports quantitatifs choisis de manière qu'il y ait pour chaque groupe amino 0,95 à 2,5 groupes isocyanate du polyisocyanate organique.

4. Utilisation des progéniteurs de matières plastiques porteurs de noyaux d'oxazoline-2-one pouvant être obtenus conformément aux revendications 1 à 3 pour la production de matières plastiques macromoléculaires, caractérisée en ce qu'on soumet les progéniteurs de matières plastiques, éventuellement en présence de composés qui portent au moins deux groupes aptes à réagir, au sens d'une réaction d'addition ou de condensation, avec des noyaux d'oxazoline-2-one, à un traitement thermique dans la plage de 100 à 200° C.

5. Utilisation suivant la revendication 4, caractérisée en ce qu'on utilise comme composés porteurs de groupes aptes à réagir vis-à-vis de noyaux d'oxazoline-2-one, des polyisocyanates organiques ou des polyamines organiques à groupes amino primaires et/ou secondaires.